# EUROPEAN PATENT SPECIFICATION

(11) **EP 1 223 981 B1**
(45) Date of publication and mention of the grant of the patent: **16.01.2008**
(21) Application number: 00973678.6
(22) Date of filing: 19.10.2000
(51) Int. Cl.: A61K 39/395, A61P 27/02, C07K 16/28, A61K 31/00, A61K 38/13

(54) **USE OF A CD40:CD154 BINDING INTERRUPTOR TO TREAT IMMUNOLOGICAL COMPLICATIONS OF THE EYE**
VERWENDUNG EINES CD40:CD154 BINDUNGS-UNTERBRECHERS ZUR BEHANDLUNG IMMUNOLOGISCHER KOMPLIKATIONEN DES AUGES
UTILISATION D'UN INTERRUPTEUR DE LIAISON CD40:CD154 POUR LE TRAITEMENT DE COMPLICATIONS IMMUNOLOGIQUES DE L'OEIL

(30) Priority: 22.10.1999 US 160909 P; 11.04.2000 US 196453 P; 31.08.2000 US 229491 P
(43) Date of publication of application: 24.07.2002
(73) Proprietor: Biogen Idec MA Inc., Cambridge, Massachusetts 02142 (US); THE SCHEPENS EYE RESEARCH INSTITUTE, Boston, MA 02114 (US)
(72) Inventor: DANA, M., Reza, Belmont, MA 02478 (US); VAISHNAW, Akshay, K., Arlington, MA 02474 (US); BURKLY, Linda, C., West Newton, MA 02165 (US); LOBB, Roy, Westwood, MA 02090 (US); ADELMAN, Burt, Concord, MA 01742 (US)
(74) Representative: Vossius & Partner
(86) International application number: PCT/US2000/028945
(87) International publication number: WO 2001/030386

(56) References cited:
- EP-A- 0 794 441
- WO-A-93/06865
- WO-A-98/52606
- WHITCUP S M ET AL: "Anti-CD40 ligand (CD40L) antibody inhibits experimental autoimmune uveitis (EAU)." IOVS, vol. 39, no. 4, 15 March 1998 (1998-03-15), page S867 XP002159323 Annual Meeting of the Association for Research in Vision and Ophthalmology;Fort Lauderdale, Florida, USA; May 10-15, 1998
- LARSEN C L ET AL: "CD40-gp39 interactions play a critical role during allograft rejection" TRANSPLANTATION,US,WILLIAMS AND WILKINS, BALTIMORE, MD, vol. 1, no. 61, 15 January 1996 (1996-01-15), pages 4-9, XP002074814 ISSN: 0041-1337
- YATOH S. ET AL: 'Effect of a topically applied neutralizing antibody against vascular endothelial growth factor on corneal allograft rejection of rat' TRANSPLANTATION vol. 66, no. 11, 15 December 1998, WILLIAMS AND WILKINS, BALTIMORE, MD, US, pages 1519 - 1524, XP009043386

## Description

This invention relates to the use of an anti-CD40L (anti-CD154) monoclonal antibody for the preparation of a pharmaceutical composition for inhibiting ocular neovascularization in a subject.

Organ transplantation between genetically non-identical individuals often results in immunological rejection of the organ through T cell dependent mechanisms, unless the organ is transplanted to an immunologically privileged site or the rejection process is blunted by administering drugs that suppress T cell function. Several United States patents disclose the use of such immunosuppressant drugs for inhibiting graft rejection, including United States patents 5,104,858; 5,008,246 and 5,068,323. Other conventional agents are described in Suthanthiran et al. (1994), 331 New Eng. Med. J. 365-376. Both calcineurin phosphatase inhibitors and glucocorticosteroids are used clinically, and both prevent the T cell mediated release of activating cytokines, particularly IL-2. However, therapy with these types of conventional agents remains imperfect. Both calcineurin phosphatase inhibitors and glucocorticosteroids act by impairing signaling through the T cell antigen receptor (TCR), the sole mediator of T cell antigen specificity, and act on all T cells indiscriminately (pan-immunosuppression). In addition, since the effect of these drugs is not lasting, cessation of treatment generally results in graft loss. Thus, to maintain viable, functional integration of the graft, transplant recipients must suffer the consequences of long-term, non-specific immunosuppression. These consequences include an increased risk of infection and malignancy, as well as significant toxicity and economic expense.

The eye, in terms of immunology of grafts, inflammation and other immunological complications such as ocular angiogenesis (e.g., neovascularization), differs from solid organs in that it is immunologically privileged. That is, in the absence of injury or inflammation, the eye is devoid of bone-marrow derived immune system cells. Allografts of solid organs sensitize T cells via the direct pathway, i.e., host T cells become activated by antigen presenting cells from the donor tissue; in contrast, allografts in the eye sensitize T cells mainly via the indirect pathway, in which the recipient (host or subject) antigen presenting cells process alloantigen and sensitize host T cells, which in turn initiate the effector response at the graft site. In other words, graft antigens are presented in the context of a host antigen presenting cell surface. However, in allografts of high-risk corneal transplant recipients, the direct sensitization route (involving antigen presentation in the context of graft-derived antigen presenting cells) may be involved as well. Ocular angiogenesis (e.g., ocular neovascularization) is undesirable because the new blood vessels bring the immune system to the eye. Accordingly, treatment using immunomodulatory agents in the eye cannot be predicted by preclinical studies involving other organs, tissues and cells.

There are close to 45,000 corneal allograft transplants performed each year in the United States, far more than all other types of allografts combined. The overall rejection rate of corneal transplant is about 20-30% and approximately half of these are reversible with anti-inflammatory and immunosuppressive therapy. The overall failure rate in normal-risk cases is about 10%, whereas the failure rate in high-risk cases - patients with a prior history of unsuccessful corneal transplantation, patients with a neovascularized corneal host bed or patients with a history of ocular inflammatory disease, such as uveitis or keratitis - can range between about 50% and about 90%. These data do not take into account that many high-risk candidates never receive corneal transplants because of a surgeon's judgment that the grafts will be swiftly rejected. Furthermore, data from the Eye Bank Association of America clearly show that an increasing proportion of corneal graft recipients each year are high-risk recipients who are being re-grafted due to prior graft rejection. Additionally, the topical and systemic immunosuppressive regimens being used for inhibiting corneal transplant rejection are uniformly toxic. For example, corticosteroids lead to cataracts and glaucoma and opportunistic infections in many patients, even those receiving the corticosteroids topically.

Ocular inflammatory diseases are potentially debilitating. The administration of pan-immunosuppressants to patients suffering from an ocular inflammatory disease generally does not substantially alleviate the symptoms and results in high toxicity.

Another class of potentially debilitating illnesses of the eye involves retinal degeneration, such as age-related macular degeneration. A possible treatment for age-related macular degeneration that is currently being investigated is transplantation of retinal pigment epithelial cells or tissue. Other illnesses of the eye that can be treated by allograft or xenograft of retinal cells include glaucoma, retinitis pigmentosa and diabetic retinopathy. These grafts should be accompanied by immunosuppressant therapy. Conventional pan-immunosuppressants, however, are not very effective in inhibiting ocular graft rejection and are quite toxic.

Ocular angiogenesis, including ocular neovascularization, is another condition that can be inhibited at least to some extent by immunosuppressants. However, as discussed previously, currently available pan-immunosuppressants are not very effective and are quite toxic, thereby limiting their utility.

There is accordingly a need for improved or more effective immunosuppressive or immunomodulatory treatments for the above-described conditions and illnesses. In particular, there is a need for treatments that do not require pan-T cell immunosuppression, i.e., treatments that do not leave the recipient vulnerable to malignancies or opportunistic infection. More pointedly, there is a need for treatments that have less toxicity than conventional therapeutic agents. Similarly, there is a need for treatments that promote lasting functional integration of the graft, i.e., integration that persists beyond termination of the course of treatment.

The present invention discloses the use of an anti-CD40L (anti-CD154) monoclonal antibody for the preparation of a pharmaceutical composition for inhibiting ocular neovascularization in a subject.

The specification also describes a method of treating or inhibiting ocular inflammatory disease in a subject (i.e., patient) by administering to the subject an immunomodulatory agent which does not lead to pan-T cell immunosuppression.

This specification also describes a method to treat, prevent, or inhibit rejection of an ocular graft (e.g., a corneal graft or a retinal graft) in a subject (i.e., a graft recipient) receiving said graft by administering to the subject an immunomodulatory agent in the absence of pan-T cell immunosuppression.

This specification also describes methods to reverse rejection of an ocular graft, including a corneal graft and a retinal graft, in a subject receiving said ocular graft (i.e., the ocular graft recipient); methods to prolong survival of ocular graft in a subject receiving said ocular graft (i.e., the graft recipient), or methods to attenuate immunological complications of failure of an ocular graft in a subject receiving said ocular graft (i.e., ocular graft recipient) by administering to the subject an immunomodulatory agent without leading to pan-T cell immunosuppression.

This specification also describes a method for inhibiting ocular angiogenesis, particularly ocular neovascularization, in a subject (i.e., patient) by administering to the subject an immunomodulatory agent that does not produce pan-T cell immunosuppression. Also described is an immunomodulatory agent that promotes functional integration of an ocular graft (e.g., a corneal or a retinal graft) in a subject receiving said graft (i.e., a recipient). Further described is an immunomodulatory agent that inhibits immunological rejection of tissue or cells grafted into a retinal or corneal site and an immunomodulatory agent that interrupts delivery of a costimulatory signal to activated T cells, particularly T cells that are activated by ocular tissue-specific antigens.

This description also relates to a contact lens, an eye wash solution, an eye ointment, an eye drop solution, an intravitreal insert and an eye shield for treating or inhibiting an ocular inflammatory disease in a subject (i.e., patient), or treating, inhibiting, or reversing rejection of an ocular graft by a subject receiving said ocular graft (i.e., an ocular graft recipient). The invention provides a method for inhibiting ocular angiogenesis, especially neovascularization, in a subject (i.e., patient), in each case, the lens, solution, ointment, an intravitreal insert or shield comprising an effective amount of an anti-CD40L (anti-CD154) monoclonal antibody.

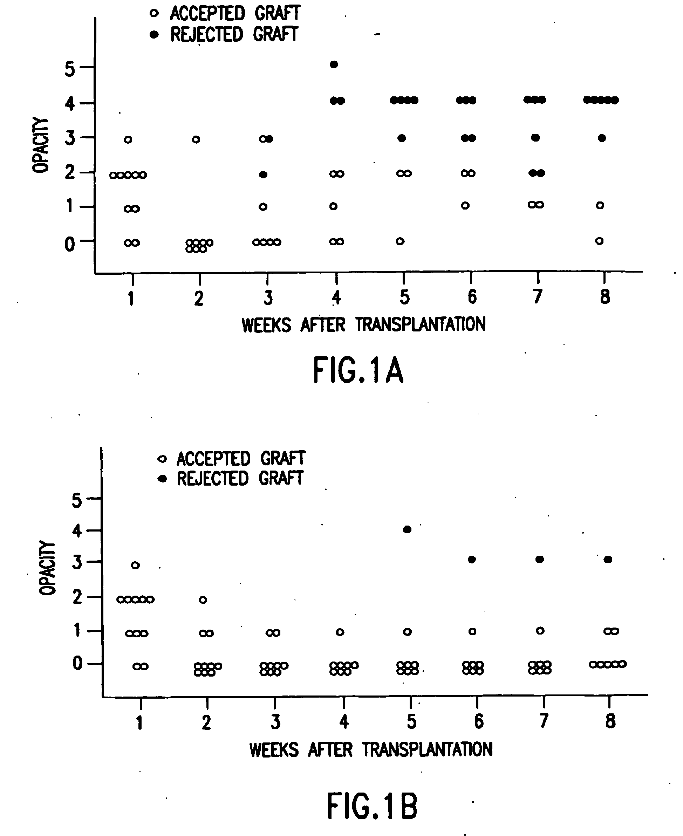

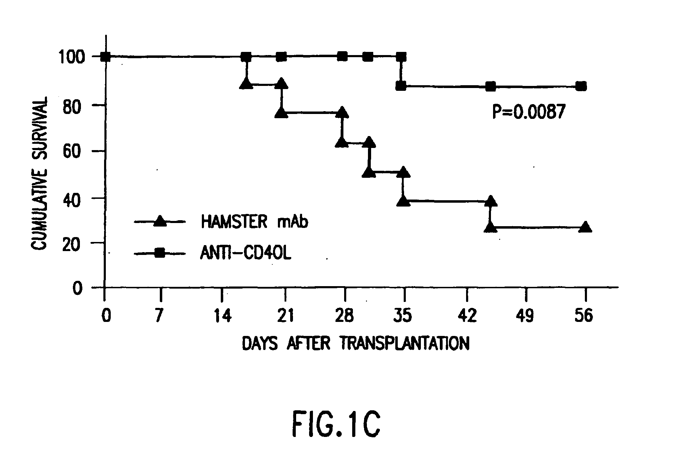

**Figure 1**. Fate of minor H-disparate corneal transplants.
   BALB/c mice (n = 10/group) received corneal transplants from B10.D2 mice, and were randomized to receive anti-CD40L mAb or control hamster monoclonal antibody (mAb). Graft opacification was evaluated clinically and scored for control hamster mAb (A)
   and anti-CD40L mAb treated animals (B).
   Graft survival data are presented as Kaplan-Meier survival curves (C). Anti-CD40L mAb treatment promotes the survival of minor H-disparate grafts (P = 0.0087).

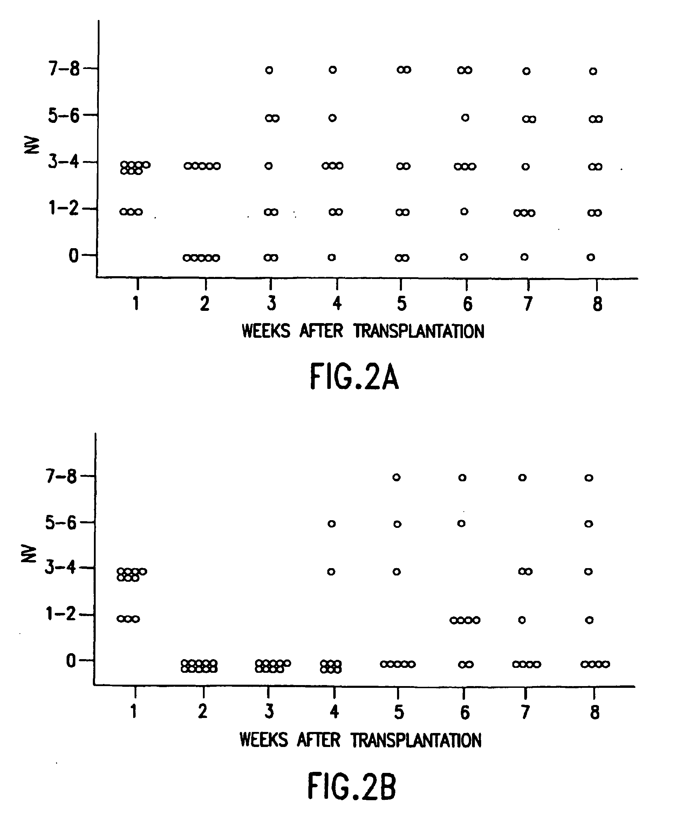

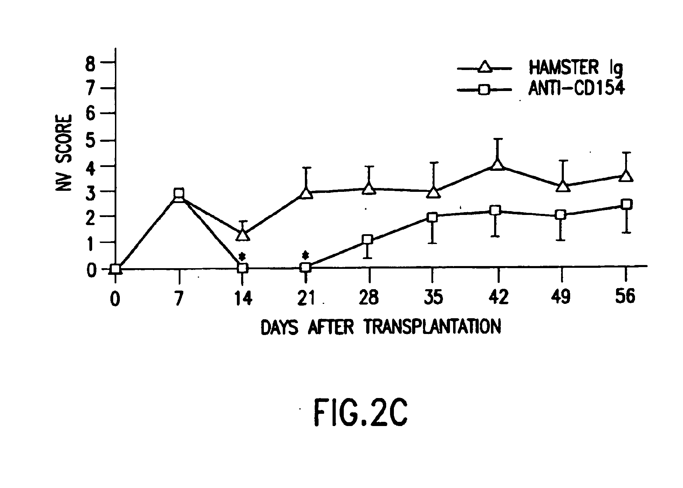

**Figure 2**. Corneal neovascularization (NV) scores in minor-H-disparate allografts treated with control hamster mAb (**A**) or anti-CD40L mAb (**B**). The mean NV scores of the results displayed in (**A**) and (**B**) in dot plot form are displays in graphical form in (**C**). Anti-CD40L mAb treatment resulted in an angiostatic effect during the early postoperative period (P= 0.0455 at week 2, P = 0.0273 at week 3).

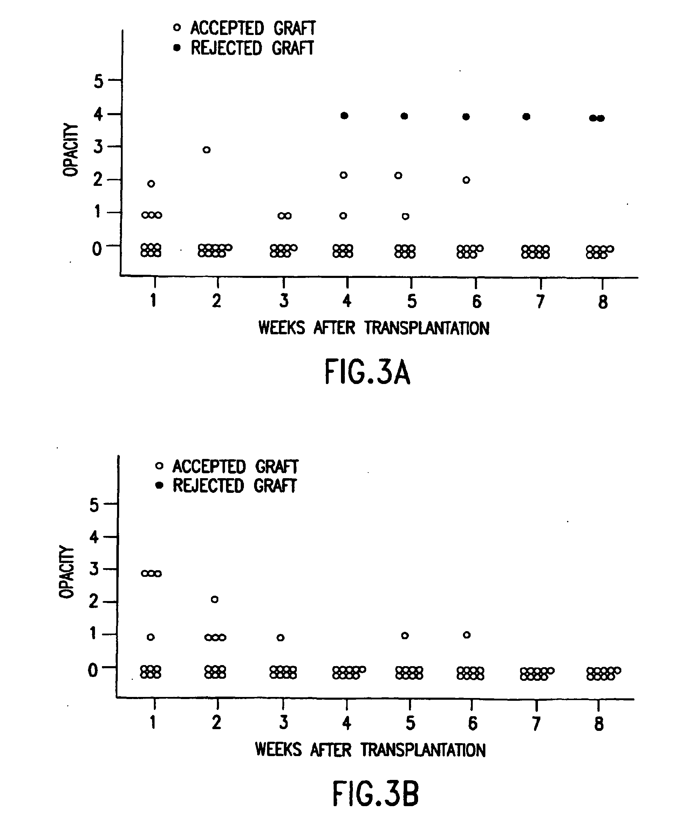

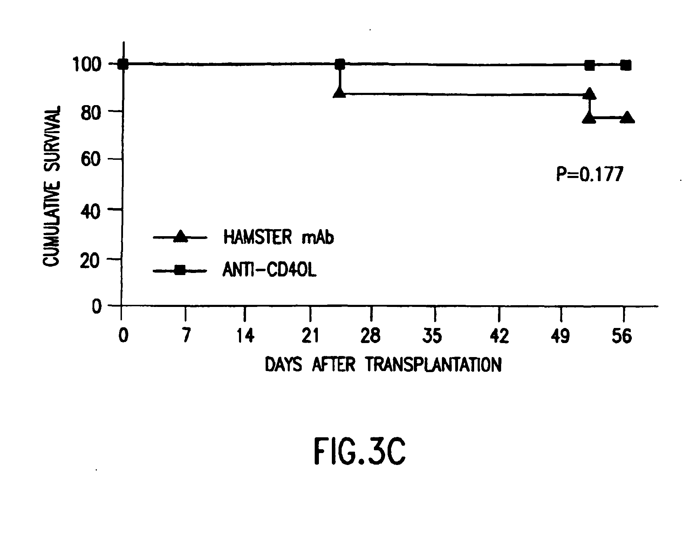

**Figure 3**. Fate of MHC-mismatched orthotopic corneal transplants. BALB/c mice (n = 10/group) received corneal transplants from BALB.b mice, and were randomized to receive anti-CD40L mAb or hamster mAb. Graft rejection was evaluated clinically and scored in control hamster mAb (**A**) and anti-CD40L mAb treated (**B**) mice. Graft survival data are presented as Kaplan-Meier survival curves (**C**). There was universal survival of MHC-disparate allografts in subjects treated with anti-CD40L mAb (P = 0.177).

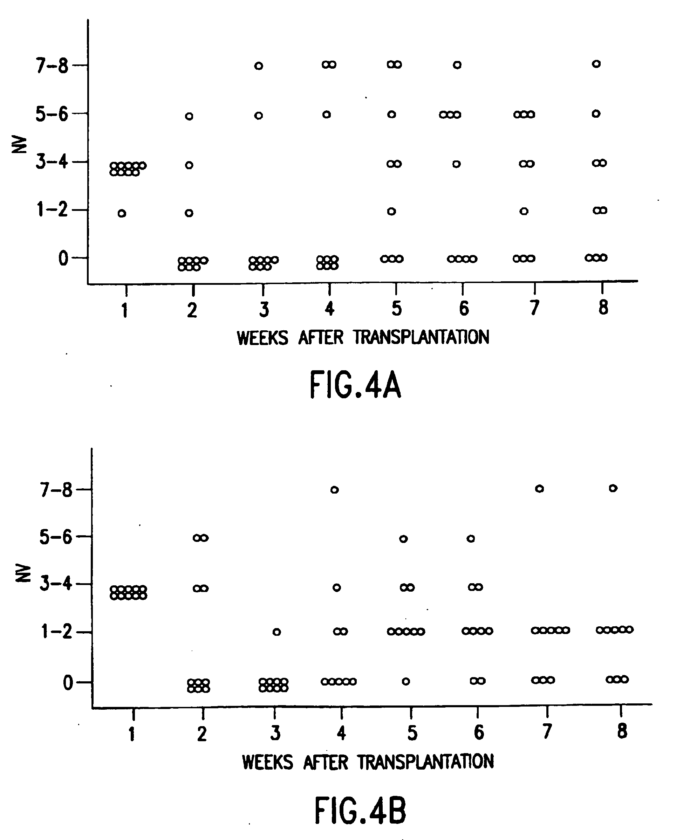

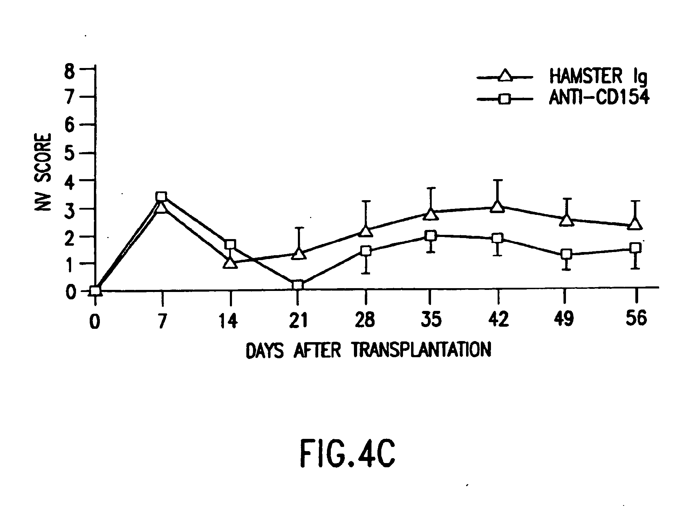

**Figure 4**. Corneal neovascularization (NV) in MHC-mismatched allografts treated with control hamster mAb (**A**) or anti-CD40L mAb (**B**). The mean NV scores of the results displayed in (**A**) and (**B**) in dot plot form are displays in graphical form in (**C**). Anti-CD40L mAb therapy imposed no appreciable effect on postkeratoplasty angiogenesis.

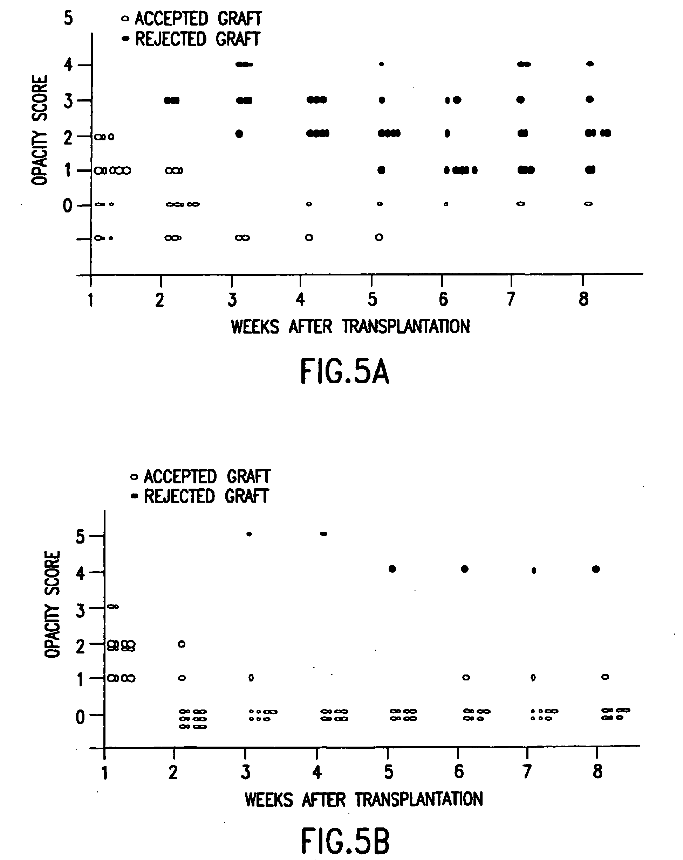

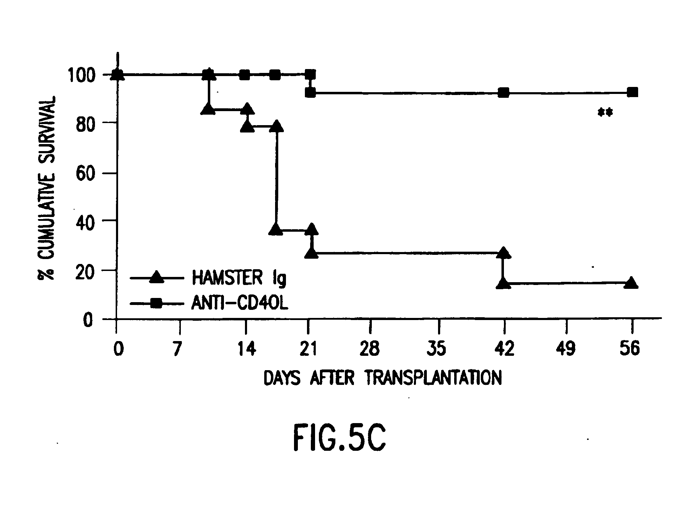

**Figure 5**. Fate of high-risk MHC-mismatched orthotopic corneal transplants placed into vascularized recipient beds. BALB/c mice (n = 14/group) were induced to grow neovessels in their corneas by three stromal sutures. Two weeks later, the mice received corneal transplants from BALB.b mice, and were randomized to receive anti-CD40L mAb or hamster mAb (control mAb). Graft rejection was evaluated clinically and scored in control mAb (**A**) and anti-CD40L mAb treated mice (**B**). Graft survival data are presented as Kaplan-Meier survival curves (**C**). Anti-CD40L mAb therapy enhances the survival of MHC-disparate grafts in high-risk transplantation (P = 0.0002).

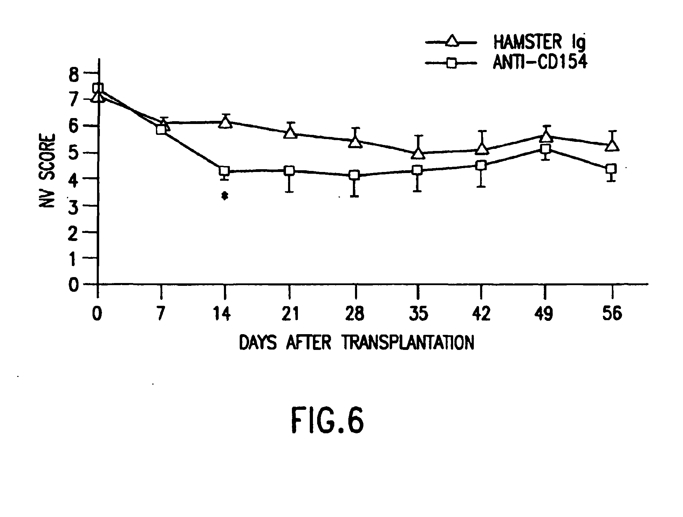

**Figure 6.** Corneal neovascularization in MHC-mismatched allografts which were placed into high-risk vascularized beds and treated with control hamster Ig. Hosts treated with anti-CD40L mAb displayed less neovascularization during week 2 (P = 0.0152).

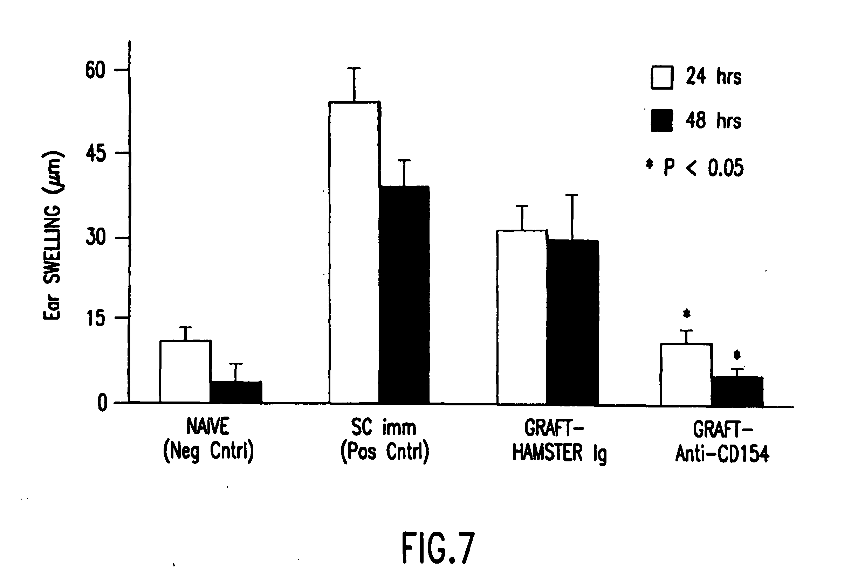

**Figure 7.** Donor-specific delayed hypersensitivity in BALB/c mice bearing orthotopic BALB.b corneal allografts 3 weeks after high-risk transplantation. Ear swelling of naïve, subcutaneously primed (positive control), control hamster Ig-treated, and anti-murine-CD40L mAb treated mice were measured by a micrometer 24 and 48 hours after ear challenge. Anti-CD40L mAb therapy significantly inhibited the degree of ear swelling compared with positive control or hamster control Ig treatment (P < 0.05).

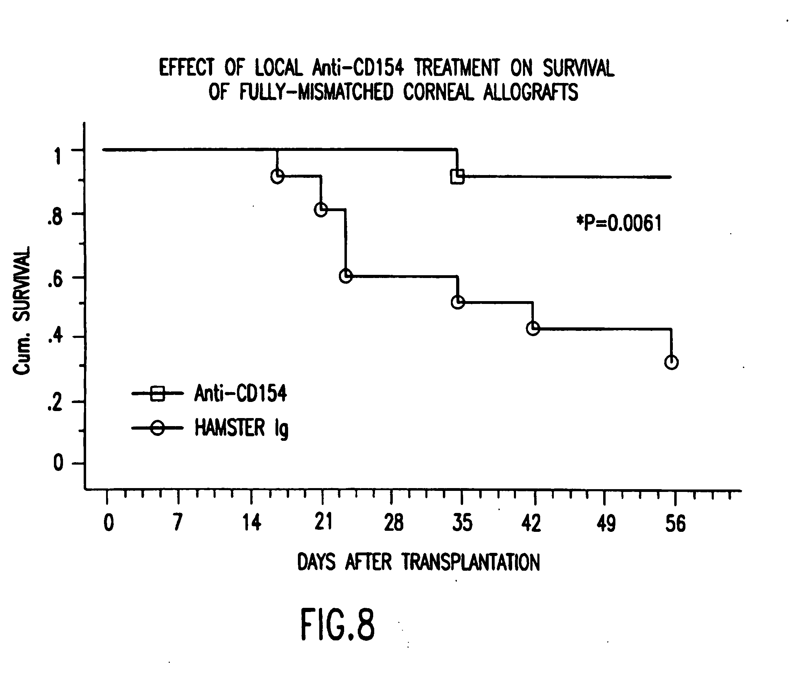

**Figure 8.** Effect of local anti-CD40L mAb treatment on survival of fully-mismatched corneal allografts in a normal-risk host model.

Data establishing that T cell activation requires both TCR mediated signals and simultaneously delivered costimulatory signals have accumulated over the past twenty years. For example, antibody production by B lymphocytes in response to protein antigens requires a specific, costimulatory interaction with T lymphocytes. This B cell/T cell interaction is mediated through several receptor-ligand binding events in addition to engagement of the TCR. These additional binding events include the binding of CD40 on B cells to CD154 (CD40L) on T cells. Human CD40 is a 50 kilodalton cell surface protein expressed on mature B cells, as well as macrophages and activated endothelial cells. CD40 belongs to a class of receptors involved in programmed cell death, including Fas/CD95 and the tumor necrosis factor (TNF) alpha receptor. Human CD154 (CD40L) is a 32 kilodalton type II membrane glycoprotein with homology to TNF alpha that is transiently expressed primarily on activated T cells. CD40:CD154 binding has been shown to be required for essentially all T cell-dependent antibody responses. In particular, CD40:CD154 binding provides anti-apoptotic and/or lymphokine stimulatory signals.

Another important costimulatory signal is produced by the binding of CD28 on T cells to its counter receptor CD80 (B7-1) or CD86 (B7-2) on antigen presenting cells (APCs) and perhaps also on parenchymal cells. Significantly, CD80 and/or CD86 expression is upregulated by signals initiated on the binding of CD40 to CD154. Further studies have shown that the T cell molecule CTLA4 (CD152) appears to down-regulate costimulation and TCR mediated activation, at least in part by competing with CD28 for CD80/CD86, and by delivering a unique negative signal to the TCR signal transduction complex.

A number of preclinical studies, including those described in co-pending, commonly assigned PCT patent applications published as W098/30241, W098/30240, W098/52606, W098/58669 and W099/45958, have established that agents capable of interrupting CD40:CD154 binding have promise as immunomodulating agents. WO 98/52606 further describes the use of CD40:CD154 binding interrupters, e.g., 5c8 antibody to treat graft rejection. The method is exemplified for renal allografts. It is further disclosed that the interrupter can be administered alone or in combination with another immunosuppressant or immunomodulator. In murine systems, antibodies to CD154 (CD40L) block primary and secondary immune responses to exogenous antigens, both in vitro and in vivo. Antibodies to CD154 cause a reduction in germinal centers in mice and monkeys, consistent with data on CD154 immunodeficiency.

Administration of three doses of anti-CD154 antibody to lupus-prone mice, aged three months, substantially reduced titers against double-stranded DNA and nucleosomes, delayed the development of severe nephritis, and reduced mortality. Moreover, administration of anti-CD154 antibodies to mice aged five to seven months with severe nephritis was shown to stabilize or even reverse renal disease. Anti-CD154 antibodies given concomitantly with small resting allogeneic lymphocytes permitted unlimited survival of mouse pancreatic islet grafts. In other animal models, interference with CD40:CD154 binding has been demonstrated to reduce symptoms of autoimmune disease (e.g., multiple sclerosis, rheumatoid arthritis, inflammatory bowel disease), graft rejection (cardiac allograft, graft-versus-host disease), and mercuric chloride induced glomerulonephritis, which is mediated by both humoral and cellular mechanisms.

Further, Larsen et al. (Transplantation, Vol. 1, No. 61, page 4-9 (1996)) reports on the role of CD40-gp39 interactions during allograft rejection in a heart transplantation model.

Additional studies in rodents have shown that T cell activation can be blocked, and rodent allograft survival prolonged, by interfering with the binding of CD80/CD86 to its T cell counter receptors, CD28 and CTLA4. These studies involved the use of the CD80/CD86 specific fusion protein, CTLA4-Ig, as a CD28 signaling interruptor. Others have demonstrated that CD80/CD86 up-regulation can be prevented by use of a CD40:CD154 binding interruptor. Both classes of immunomodulatory agents appear to be dependent on TCR engagement for their effectiveness. Thus, such agents offer the capacity to modulate the specificity of T cell dependent biological processes, rather than depending on pan T cell immunosuppression. Studies involving the use of such agents in vivo in rodent models of graft rejection have produced dramatic results, including the acceptance of fully mismatched skin grafts, a result not obtainable with currently available immunosuppression. Yatoh S. et al (Transplantation, Vol 66 No 11, page 1519-1524 (1998) discloses the use of anti-VEGF antibody for the treatment of corneal transplantation. Anti-VEGF antibody inhibited the neovascularization.

The present invention rests on the discovery that use of an anti-CD40L (anti-CD154) monoclonal antibody as a CD40 : CD154 binding interruptor, alone or in combination with another immunomodulatory agent, inhibits ocular neovascularization. Such use is unaccompanied by pan-suppression of the recipient's immune system.

The description provides methods and compositions for immunomodulatory therapy for subjects (i.e., recipients) receiving grafted corneal or retinal tissue or cells. One method treats, inhibits or reverses rejection of a corneal or retinal tissue or cell graft by a graft recipient (i.e., subject) by administering to the graft recipient a CD40:CD154 (CD40L) binding interruptor. Another method treats or inhibits ocular inflammatory diseases. A first method according to the present invention treats or inhibits ocular angiogenesis, particularly ocular neovascularization.

In one aspect of the specification, the immunomodulatory agent is a CD40:CD154 binding interruptor. In one embodiment, the CD40:CD154 binding interruptor is an anti-CD40L (anti-CD154) compound. In an even more preferred embodiment, the anti-CD40L compound is a monoclonal antibody or a derivative thereof, such as a humanized antibody. CD40:CD154 binding interruptors useful in the invention include any agent that interrupts the binding of a costimulatory molecule (here, CD40 ligand, also referred to herein as the 5c8 antigen, T-BAM, CD40L, CD154 and also referred to in the art as gp39 or CD40CR) to its counter or cognate receptor (here, CD40). Preferably, the binding interruptor is an anti-CD40L compound, by which is meant a compound that binds to CD40L (CD154) and thereby blocks, interferes with or disrupts the ability of CD40L to bind to CD40. An exemplary anti-CD40L compound is a monoclonal antibody, particularly an antibody having the antigen-specific binding characteristics of the 5c8 antibody (produced by ATCC Accession Number HB 10916) disclosed in United States patent 5,474,771.

A particular aspect of the specification is a CD40:CD154 binding interruptor for use in therapy, particularly for use in therapy to mitigate or delay immunological rejection of grafted corneal or retinal tissue or cells. The present invention relates to a therapy to treat or inhibit ocular angiogenesis, particularly ocular neovascularization. Another aspect is a therapeutic composition and treatment regime for mitigating corneal or retinal graft rejections, or ocular inflammatory diseases. The present invention provides a therapeutic composition for pathologic ocular angiogenesis (e.g., ocular neovascularization), based on the use of an anti-CD40L (anti-CD154) monoclonal antibody alone or in combination with another immunosuppressant or immunomodulator. A specific object of the invention is to provide a therapeutic composition and treatment regime based on the use of an anti-CD40L (anti-CD154) monoclonal antibody in combination with an agent that blocks costimulation via CD28, CD80 or CD86. A more general aspect of the Specification is a therapeutic composition and treatment regime for inhibiting, mitigating, attenuating, delaying or reversing failure or acute rejection of grafted corneal or retinal tissue or cells. Another general aspect of the description is to improve the availability of corneal or retinal tissue grafts, by teaching immunomodulatory compositions that allow functional integration of allogeneic or xenogeneic tissue into a recipient host (i.e., subject).

One method of this specification prolongs survival of a corneal or retinal tissue or cell graft in a graft recipient (i.e., subject), by treating the graft recipient with a CD40:CD154 binding interruptor, preferably with an anti-CD40L monoclonal antibody. Another method of this specification attenuates immunological complications of failure of grafted corneal or retinal tissue or cells, by treating a graft recipient (i.e., subject) with a CD40:CD154 binding interruptor, preferably with an anti-CD40L monoclonal antibody. That is, the method inhibits, suppresses, mitigates or detectably decreases such immunological complications.

A method described in the specification prevents onset of or delays progression of ocular angiogenesis, specifically ocular neovascularization, in a subject (i.e., patient) by treating the subject with a CD40:CD154 binding interruptor, preferably with an anti-CD40L monoclonal antibody. Another method described in the specification attenuates immunological complications of ocular inflammatory diseases, particularly ocular neovascularization, in a subject (i.e., patient) by treating a subject with a CD40:CD154 binding interruptor, preferably with an anti-CD40L monoclonal antibody. That is, the method inhibits, suppresses, mitigates or detectably decreases such immunological complications.

The foregoing methods thus are effective for treatment of acute and/or chronic rejection of grafted corneal or retinal tissue or cells and for complications associated with graft failure or ocular diseases. These methods can be used prophylactically, for post-operative treatment, or for reversing or suppressing graft rejection or disease at any time during the subject's lifetime.

Disclosed proof-of-principle studies of this specification, in contrast to conventional methods, establish that use of a CD40:CD154 binding interruptor promotes long-term, rejection free integration of heterologous (MHC-mismatched) donor corneal tissue into a subject (i.e., graft recipient). It is encouraging that the therapy described herein was tolerated remarkably well by the recipients, with no sign of premature death or secondary infections.

The following discussion illustrates and exemplifies the variety of contexts and circumstances in which the invention can be practiced, as well as providing proof-of-principle studies involving specific embodiments of the invention.

### Ocular Inflammatory Disease

The specification describes methods and compositions for treating or inhibiting ocular inflammatory diseases comprising the step of administering an effective amount of a CD40 : CD154 binding interruptor to a subject (i.e., patient) having one or more ocular inflammatory diseases. Ocular inflammatory diseases include, inter alia, uveitis, keratitis, intra-ocular inflammation, allergy and dry-eye syndrome. Keratitis may include, inter alia, immune keratitis and infectious keratitis. One example of infectious keratitis is herpes keratitis. One example of dry-eye syndrome is Sjorgren's syndrome.

Uveitis, which is inflammation of the uveal tract, may either be a primary ocular disease or reflect ocular involvement secondary to a systemic disease. In this regard it has to be mentioned that an anti-CD40L monoclonal antibody interrupting CD40:CD154 binding, to treat experimental autoimmune uveitis (EAU) has been described by Whitcup et al. (IOVS, Vol. 39, No. 4, S867; 1998). A large number of systemic diseases, including various forms of collagen vascular disease, lymphoma, and infectious diseases such as AIDS, could result in presentation of uveitis. Uveitis may also be classified according to site of inflammation. For example, anterior uveitis refers to inflammation confined primarily to the iris and the anterior chamber of the eye. See, e.g., Power, W.J., Principles and Practices of Opthalmology 2nd Ed. Ch. 86, pp. 1189-1197 and Foster C.S. et al. Principles and Practices of Opthalmology 2nd Ed. Ch. 87, pp. 1198-1217 (Albert and Jacobiec, editors; published by Saunders Company) (2000).

### Donor or Graft Tissue

The specification relates to any type of eye tissue transplant or graft procedure, particularly procedures wherein the donor (i.e., grafted) tissue is affected by, or at risk of, failure or rejection by the recipient host's (i.e., subject's) immune system. In particular, the described procedure can be used in any context wherein the donor tissue is not histocompatible with the recipient host (i.e., subject receiving the ocular graft). Thus, in addition to autologous or syngeneic donor tissue, the described procedure can be used with allogeneic or even xenogeneic donor tissue. The donor tissue can be derived, by conventional means, from a volunteer or other living donor, or from a cadaveric donor. Preferably, the donor is as histocompatible as practicable with the recipient host. Thus, where the recipient host is a human, autologous and allogeneic donor tissue is preferred. However, the donor tissue can be obtained from a heterologous species (in which case it is referred to as a heterograft or a xenograft), such as a non-human primate (e.g., a chimpanzee or a baboon), or another relatively compatible mammal (e.g., a pig).

More specifically, the present donor tissue or cells suitable for ocular grafts comprise corneal tissue or cells, or retinal tissue or cells. Alternatively, the donor tissue or cells comprise stem cells or a tissue source of stem cells, such as limbal stem cells or retinal stem cells. Appropriate stem cells can be derived from developing neural tissue (e.g., neuro-epithelium, midbrain, hippocampus or neural crest cells) or adult neural tissue (e.g., hippocampus). The stem cells are selected from the group consisting of neural stem cells, neural retinal stem cells, neuro-epithelial stem cells, limbal stem cells and hippocampal stem cells. The donor retinal cells may comprise retinal pigment epithelial cells or neural retinal cells, stem cells appropriate for producing retinal replacement tissue or a mixture of cell-types. In the case of anterior segment structure of the eye, such as the cornea, the donor cells or tissue can optionally comprise limbal stem cells or any other stem or differentiated cell type appropriate for resurfacing the eye.

The donor tissue may comprises retinal cells, particularly isolated or suspended cells, including cells withdrawn or excised from a donor host, cells maintained in primary culture, or an immortalized cell line. Each of these sources also can provide appropriate stem cell populations.

The donor tissue can include cells harboring exogenous genetic material, such as transfected or transformed host cells which have been (or are derived from ancestor cells which have been) engineered to include genetic material necessary for the production of a polypeptide of therapeutic value to the recipient host. The donor tissue can be derived from a transgenic mammal that has been engineered to include genetic material necessary for the production, in some or all of its body tissues, of a polypeptide of therapeutic value to the recipient host. Exemplary polypeptides of therapeutic value to the recipient include: hormones such as insulin or growth hormone; cytokines; growth and differentiation factors; enzymes; structural proteins; and the like.

In aspects in which the grafted tissue is a corneal graft, patients with a prior history of unsuccessful corneal transplantation, with a pathologically neovascularized host bed or those with a history of ocular inflammatory disease such as uveitis or keratitis are considered high-risk patients. Normal-risk patients are all other patients.

In aspects in which the graft is a retinal cell graft, the retinal cell graft may be, inter alia, a retinal pigment epithelial cell graft, a neural retinal cell graft, or a population of stem cells capable of differentiating into either or both of these tissues; in any case, the graft is effective in treating, for example, retinal degeneration, including macular degeneration, especially age-related macular degeneration, and diabetic retinopathy. In still other aspect of the specification the graft may be a neural retinal cell graft, which is expected to be effective in treating glaucoma.

### Ocular Graft Versus Host Disease

One manifestation of graft versus host disease (wherein the grafted tissue is any tissue, organ or cells, for example, bone marrow) is an ocular graft versus host disease, typically characterized by ocular inflammation. This description specifies methods to treat or inhibit ocular graft versus host disease by administering an effective amount of a CD40:CD154 binding interruptor to a subject suffering from or at risk of ocular graft versus host disease.

### ocular Angiogenesis

As used here in, the term "ocular angiogenesis" designates the formation of new blood vessels in the eye. As used herein, the term "ocular neovascularization" designates the unwanted formation of new blood vessels in the eye, especially at sites that interfere with vision or other normal functions of the eye.

Ocular angiogenesis is a potentially debilitating condition because newly formed blood vessels may inappropriately bring the immune system to the eye, causing a variety of immunological and inflammatory complications. This invention provides methods for treating or inhibiting such unwanted ocular angiogenesis (e.g., ocular neovascularization) by administering a CD40:CD154 binding interruptor to a patient who may have or will have ocular angiogenesis anywhere in the patient's eye, for example, in the choroidal, corneal, retinal, uveal or iris tissue. Such ocular neovascularization is, among other things, a frequent complication of corneal or retinal grafts, especially in retinal graft cases in which the graft recipient has retinal degeneration, which can be macular degeneration, which can be age-related macular degeneration. Similarly, the specification also represents a new means for treating diabetic retinopathy glaucoma or retinitis pigmentosa.

### Donor and Recipient Hosts

The methods and compositions described are suitable for use with all types of graft procedures. Thus, the invention is suitable for use where the graft recipient (subject or host) is a mammal, preferably a primate, most preferably a human. The graft donor may be a non-syngeneic member of the same phylogenetic species as the graft recipient (i.e., an allogeneic donor, providing allograft tissue), or a member of a distinct phylogenetic species (i.e., a xenogeneic donor, providing xenograft tissue). If a xenogeneic donor is used as the graft tissue source, preferably the donor is relatively MHC-compatible with the recipient host; for example, a baboon or chimpanzee would be preferred as a donor for grafting tissue into a human.

The specification also describes the treatment or prophylaxis of any mammalian recipient of an eye tissue or cell graft, or any mammal in need of an eye tissue or cell graft. The invention can be used for treatment or prophylaxis of any mammalian subject (host) who suffers from an ocular inflammatory disease, especially ocular neovascularization. Preferably, the recipient (also referred to herein as the recipient host or simply the host, as the patient or as the subject) is a primate, more preferably a higher primate, most preferably a human. In other embodiments, the host may be another mammal in need of an eye tissue or cell graft, particularly a mammal of commercial importance, or a companion animal or other animal of value, such as a member of an endangered species. Thus, hosts also include, but are not limited to, sheep, horses, cattle, goats, pigs, dogs, cats, rabbits, guinea pigs, hamsters, gerbils, rats and mice.

### Exemplary CD40:CD154 Interruptors

Therapeutic compounds useful in the methods of the invention include any compound that blocks the interaction of cell surface CD40 (e.g., on B cells, dendritic cells, endothelial cells and other antigen presenting cells) with CD40L (CD154) expressed on the surface of activated T cells. CD40:CD154 binding interruptor compounds, such as anti-CD40L compounds, that are specifically contemplated include polyclonal antibodies and monoclonal antibodies (mAbs), as well as antibody derivatives such as chimeric molecules, humanized molecules, molecules with altered (e.g., reduced) effector functions, bispecific molecules, and conjugates of antibodies. In a preferred embodiment, the antibody is 5c8 (produced by ATCC Accession Number HB 10916), as described in United States patent 5,474,771. In a highly preferred embodiment, the antibody is a humanized 5c8. Other known antibodies against CD154 include antibodies ImxM90, ImxM91 and ImxM92 (described in United States patent 5,961,974), an anti-CD40L mAb commercially available from Ancell (clone 24-31, catalog # 353-020, Bayport, MN), and an anti-CD40L mAb commercially available from Genzyme (Cambridge, MA, catalog # 80-3703-01). Also commercially available is an anti-CD40L mAb from PharMingen (San Diego, catalog #33880D). Numerous additional anti-CD40L antibodies have been produced and characterized (see, e.g., PCT patent application WO 96/23071 of Bristol-Myers Squibb). For murine preclinical studies, antibodies which specifically bind to murine CD40L should be used - an example of such an antibody is MR1 (see Noelle et al. (1992), Proc. Natl. Acad. Sci. USA 89: 6550). The selection of an appropriate monoclonal antibody (mAb) will depend on the species of the host or recipient host and the species specificity of the anti-CD40L monoclonal antibody. For example, mAb 5c8, produced by ATCC Accession No. HB 10916 and raised against human CD40L, specifically binds to human and some non-human primate but not murine CD40L and it should therefore be selected for human and selected non-human primate use and not murine use.

The invention also includes anti-CD40L molecules of other types, such as complete Fab fragments, F(ab')₂ compounds, V_{H} regions, F_{V} regions, single chain antibodies (see, e.g., PCT patent application WO 96/23071), polypeptides, fusion constructs of polypeptides, fusions of CD40 (such as CD40Ig, as in Hollenbaugh et al., J. Immunol. Meth. 188:1-7, 1995), and small molecule compounds such as small semi-peptidic compounds or non-peptide compounds, all capable of blocking or interrupting CD40:CD154 binding. Procedures for designing, screening and optimizing small molecules are provided in commonly assigned PCT patent publication WO97/00895.

Various forms of antibodies may also be produced using standard recombinant DNA techniques (Winter and Milstein, Nature 349: 293-99, 1991). For example, "chimeric" antibodies may be constructed, in which the antigen binding domain from an animal antibody is linked to a human constant domain (an antibody derived initially from a nonhuman mammal in which recombinant DNA technology has been used to replace all or part of the hinge and constant regions of the heavy chain and/or the constant region of the light chain, with corresponding regions from a human immunoglobulin light chain or heavy chain) (see, e.g., Cabilly et al., United States patent 4,816,567; Morrison et al., Proc. Natl. Acad. Sci. 81: 6851-55, 1984). Chimeric antibodies reduce the immunogenic responses elicited by animal antibodies when used in human clinical treatments.

In addition, recombinant "humanized" antibodies may be synthesized. Humanized antibodies are antibodies initially derived from a nonhuman mammal in which recombinant DNA technology has been used to substitute some or all of the amino acids not required for antigen binding with amino acids from corresponding regions of a human immunoglobulin light or heavy chain. That is, they are chimeras comprising mostly human immunoglobulin sequences into which the regions responsible for specific antigen-binding have been inserted (see, e.g., PCT patent application WO 94/04679). Animals are immunized with the desired antigen, the corresponding antibodies are isolated and the portion of the variable region sequences responsible for specific antigen binding are removed. The animal-derived antigen binding regions are then cloned into the appropriate position of the human antibody genes in which the antigen binding regions have been deleted. Humanized antibodies minimize the use of heterologous (inter-species) sequences in antibodies for use in human therapies, and are less likely to elicit unwanted immune responses. Primatized antibodies can be produced similarly using primate (e.g., rhesus, baboon and chimpanzee) antibody genes.

Another embodiment of the invention includes the use of human antibodies, which can be produced in nonhuman animals, such as transgenic animals harboring one or more human immunoglobulin transgenes. Such animals may be used as a source for splenocytes for producing hybridomas, as is described in United States patent 5,569,825.

Antibody fragments and univalent antibodies may also be used in the methods and compositions of this invention. Univalent antibodies comprise a heavy chain/light chain dimer bound to the Fc (or stem) region of a second heavy chain. "Fab region" refers to those portions of the chains which are roughly equivalent, or analogous, to the sequences which comprise the Y branch portions of the heavy chain and to the light chain in its entirety, and which collectively (in aggregates) have been shown to exhibit antibody activity. A Fab protein includes aggregates of one heavy and one light chain (commonly known as Fab'), as well as tetramers which correspond to the two branch segments of the antibody Y, (commonly known as F(ab)₂), whether any of the above are covalently or non-covalently aggregated, so long as the aggregation is capable of selectively reacting with a particular antigen or antigen family.

In addition, standard recombinant DNA techniques can be used to alter the binding affinities of recombinant antibodies with their antigens by altering amino acid residues in the vicinity of the antigen binding sites. The antigen binding affinity of a humanized antibody may be increased by mutagenesis based on molecular modeling (Queen et al., Proc. Natl. Acad. Sci. 86:10029-33, 1989; PCT patent application WO 94/04679). It may be desirable to increase or to decrease the affinity of the antibodies for CD40L, depending on the targeted tissue type or the particular treatment schedule envisioned. This may be done utilizing phage display technology (see, e.g., Winter et al., Ann. Rev. Immunol. 12:433-455, 1994; and Schier et al., J. Mol. Biol. 255:28-43, 1996). For example, it may be advantageous to treat a patient with constant levels of antibodies with reduced affinity for CD40L for semi-prophylactic treatments. Likewise, antibodies with increased affinity for CD40L may be advantageous for short-term treatments.

### Routes of Administration

The compounds of the invention may be administered in any manner which is medically acceptable. Depending on the specific circumstances, local or systemic administration may be desirable. Local administration may be, for example, by subconjunctival administration. Preferably, the compound is administered via a parenteral route such as by an intravenous, intraarterial, subcutaneous, intramuscular, intraorbital, intraventricular, intraperitoneal, subcapsular, intracranial, intraspinal, oral, enteral, topical or intranasal injection, infusion or inhalation. The compound also may be administered by implantation of an infusion pump, or a biocompatible or bioerodable sustained release implant, into the recipient host, either before or after implantation of donor tissue.

For purposes of this invention, a preferred route of administration is by local administration. A more preferred route of administration of such compounds is by topical administration. The topical administration may be by means selected from, inter alia, a contact lens, an eye wash solution, an eye ointment, an eye shield and an eye drop solution.

Applications EP-A-0 794 441 and WO 93/06865 teach the administration of antibodies in the context of eye related diseases or grafts, via various modes including contact lenses, topical drops or ointment, and, e.g. by a depot attached to the intra ocular lens.

In general, compounds of the invention are administered to the host. However, the compounds also can be administered to the donor, or to the donor tissue. For example, a compound of the invention can be included in a perfusion or preservative fluid in which the donor tissue is stored or transported prior to its integration into the recipient host. Alternatively, the compound can be included in a cell culture or cell suspension medium.

### Dosages and Frequency of Treatment

Generally, the methods described herein involve administration of the CD40:CD154 binding interruptor at desired intervals (e.g., daily, twice weekly, weekly, biweekly, monthly or at other intervals as deemed appropriate) over at least a two- or three-week period. The administration schedule is adjusted as needed to produce a detectable decrease in indicia of counter-adaptive immune responses, such as indicia of graft rejection. The present treatment regime can be repeated in the event of a subsequent episode of illness or graft rejection. In embodiments wherein the CD40:CD154 binding interruptor is an anti-CD40L monoclonal antibody, the interruptor can be administered at doses between about 0.05 mg/kg to about 50 mg/kg body weight. Preferably, the doses are between 5mg/kg body weight and 20 mg/kg body weight.

The amount of and frequency of dosing for any particular compound to be administered to a patient for a given immunological disease is within the skills and clinical judgement of ordinary practitioners of the arts, such as opthamologists (for ocular inflammatory disease, or ocular angiogenesis including ocular neovascularization) and transplant surgeons (for corneal, retinal or other ocular transplant). The general dosage and administration regime is established by preclinical and clinical trials, which involve extensive but routine studies to determine the optimal administration parameters of the compound. Even after such recommendations are made, the practitioner will often vary these dosages for different recipient hosts based on a variety of considerations, such as the individual's age, medical status, weight, sex, and concurrent treatment with other pharmaceuticals. Determining the optimal dosage and administration regime for each anti-CD40L compound used is a routine matter for those of skill in the pharmaceutical and medical arts.

Generally, the frequency of dosing may be determined by an attending physician or similarly skilled practitioner, and might include periods of greater dosing frequency, such as at daily or weekly intervals, alternating with periods of less frequent dosing, such as at monthly or longer intervals.

To exemplify dosing considerations for an anti-CD40L compound, the following examples of administration strategies are given for an anti-CD40L mAb. The dosing amounts could easily be adjusted for other types of anti-CD40L compounds. In general, single dosages of between about 0.05 and about 50 mg/kg patient body weight are contemplated, with dosages most frequently in the 1-20 mg/kg range. For acute treatment, such as before or at the time of transplantation, or in response to any evidence that graft rejection is beginning, an effective dose of a representative antibody (such as 5c8) ranges from about 1 mg/kg body weight to about 20 mg/kg body weight, administered daily for a period of about 1 to 5 days, preferably by bolus intravenous administration. The same dosage and dosing schedule may be used in the load phase of a load-maintenance regimen, with the maintenance phase involving intravenous or intramuscular administration of antibodies in a range of about 0.1 mg/kg body weight to about 20 mg/kg body weight, for a treatment period of anywhere from weekly to 3 month intervals. Chronic treatment may also be carried out by a maintenance regimen, in which antibodies are administered by intravenous or intramuscular route, in a range of about 0.1 mg/kg body weight to about 20 mg/kg body weight, with interdose intervals ranging from about 1 week to about 3 months. In addition, chronic treatment may be effected by an intermittent bolus intravenous regimen, in which between about 1.0 mg/kg body weight and about 100 mg/kg body weight of antibodies are administered, with the interval between successive treatments being from 1 to 6 months. For all except the intermittent bolus regimen, administration may also be by oral, pulmonary, nasal, subcutaneous or subconjunctival routes.

According to an alternate aspect of the description for inhibition of graft rejection, the antibodies may be administered serially or in combination with conventional anti-rejection therapeutic agents or drugs such as, for example, corticosteroids or immunosuppressants. Alternatively, the antibodies may be conjugated to a conventional agent. This advantageously permits the administration of the conventional agent in an amount less than the conventional dosage, for example, less than about 50% of the conventional dosage, when the agent is administered as monotherapy. Accordingly, the occurrence of many side effects associated with that agent should be avoided.

For treatment, the CD40:CD154 binding interrupter can be formulated in a pharmaceutical or prophylactic composition which includes, respectively, a pharmaceutically or prophylactically effective amount of the CD40:CD154 binding interruptor dispersed in a pharmaceutically acceptable carrier. In some embodiments, the pharmaceutical or prophylactic composition can also include a pharmaceutically or prophylactically effective amount of another immunosuppressive or immunomodulatory compound, including without limitation: an agent that interrupts T cell costimulatory signaling via CD28 (e.g., CTLA4-Ig), CD80 or CD86; an agent that interrupts calcineurin signaling (e.g., cyclosporin, a macrolide such tacrolimus, formerly known as FK506); a corticosteroid; or an antiproliferative agent (e.g., azathioprine). Other therapeutically effective compounds suitable for use with the CD40:CD154 binding interruptor include rapamycin (also known as sirolimus); mycophenolate mofetil (MMF), mizoribine, deoxyspergualin, brequinar sodium, leflunomide, azaspirane and the like.

Combination therapies according to this invention for treatment or inhibition of ocular neovascularization include the use of anti-CD40L antibodies together with agents targeted at B cells, such as anti-CD19, anti-CD28 or anti-CD20 antibody (unconjugated or radiolabeled), IL-14 antagonists, LJP394 (LaJolla Pharmaceuticals receptor blocker), IR-1116 (Takeda small molecule) and anti-Ig idiotype monoclonal antibodies. Alternatively, the combinations may include T cell/B cell targeted agents, such as CTLA4Ig, IL-2 antagonists, IL-4 antagonists, IL-6 antagonists, receptor antagonists, anti-CD80/CD86 monoclonal antibodies, TNF, LFA1/ICAM antagonists, VLA4/VCAM antagonists, brequinar and IL-2 toxin conjugates (e.g., DAB), prednisone, anti-CD3 MAb (OKT3), mycophenolate mofetil (MMF), cyclophosphamide, and other immunosuppressants such as calcineurin signal blockers, including without limitation, tacrolimus (FK506). Combinations may also include T cell targeted agents, such as CD4 antagonists, CD2 antagonists and anti-IL-12 antibodies.

The immunomodulatory compound that may be co-administered with an CD40:CD154 binding interruptor to a patient with an ocular inflammatory disease, ocular angiogenesis (e.g., ocular neovascularization) or a graft recipient may be an antibody that specifically binds to a protein selected from the group consisting of CD45, CD2, IL2R, CD4, CD8 and RANK Fc.

For maintenance of graft integration, or in a period following suppression of an acute episode of graft rejection, a maintenance dose of anti-CD40L antibodies, alone or in combination with a conventional anti-rejection agent may be administered, if necessary. One or both medications may be given locally or systemically as deemed appropriate. Subsequently, the dosage or the frequency of administration, or both, may be reduced. Where no sign of graft rejection is evident, treatment might cease, with vigilant monitoring for signs of graft rejection. In other instances, as determined by the ordinarily skilled practitioner, occasional treatment might be administered, for example at intervals of four weeks or more. Recipient hosts may, however, require intermittent treatment on a long-term basis upon any recurrence of disease symptoms.

It is appreciated in the field of immunology that the administration to the host of a CD40:CD154 binding interruptor just prior to, concurrently with or shortly after transplantation result in inhibition of a counter-adaptive immune response to the antigens present on the transplanted organ, tissue or cells. Thus, the invention provides prophylactic, perioperative and treatment modalities for management of the ophthalmic conditions and diseases discussed herein.

### Formulation

In general, compounds of the invention are suspended, dissolved or dispersed in a pharmaceutically acceptable carrier or excipient. The resulting therapeutic composition does not adversely affect the recipient's homeostasis, particularly electrolyte balance. Thus, an exemplary carrier comprises normal physiologic saline (0.15M NaCl, pH 7.0 to 7.4). Other acceptable carriers are well known in the art and are described, for example, in Remington's Pharmaceutical Sciences, Gennaro, ed., Mack Publishing Co., 1990. Acceptable carriers can include biocompatible, inert or bioabsorbable salts, buffering agents, oligo- or polysaccharides, polymers, viscoelastic compound such as hyaluronic acid, viscosity-improving agents, preservatives, and the like.

An anti-CD40L compound used in the methods of the invention is administered in a pharmaceutically effective, prophylactically effective or therapeutically effective amount, which is an amount sufficient to produce a detectable, preferably medically beneficial effect on a host at risk or afflicted with corneal or retinal graft rejection, or at risk or afflicted with ocular inflammatory disease, particularly one associated with ocular neovascularization. Medically beneficial effects include preventing, inhibiting, reversing or attenuating deterioration of, or detectably improving, the host's medical condition.

### Compositions (Manufactured Articles)

This invention also provides compositions comprising a CD40:CD154 interruptor. Such compositions may be manufactured articles. The compositions, include, inter alia, a contact lens, an eye wash solution, an eye shield, an eye ointment, an intravitreal insert and an eye drop solution. The contact lens or the eye shield may be infused, coated, cross-linked or saturated with a pharmaceutically effective amount of the CD40:CD154 binding interruptor for treating or inhibiting an ocular inflammatory disease in a subject (i.e., patient) treating, inhibiting, or reversing rejection of an ocular graft (e.g., retinal graft or corneal graft) by a subject receiving said ocular graft recipient; (i.e., an ocular graft) or inhibiting ocular angiogenesis, particularly neovascularization, in a subject. The contact lens, eye shield, eye ointment, eye drop solution, intravitreal insert or the eye wash solution may comprise a pharmaceutically effective amount of the CD40:CD154 binding interruptor for treating or inhibiting an ocular inflammatory disease in a subject (i.e., patient); treating, inhibiting, or reversing rejection of an ocular graft (corneal graft or retinal graft) corneal graft by a subject receiving said ocular graft (i.e., ocular graft recipient); inhibiting ocular angiogenesis, particularly ocular neovascularization, in a subject.

In a preferred embodiment, the eye ointment is a gel. In another preferred embodiment, the eye ointment is a polymer. In a more preferred embodiment, the eye ointment is a polymer that comprises sodium hyaluronate.

In another preferred embodiment, the eye shield further comprises collagen.

In another preferred embodiment, the intravitreal insert is a slow-release insert. In another preferred embodiment, the intravitreal insert is made of polymers. A person skilled in the art would readily appreciate that such inserts are currently used to deliver gancyclovir to AIDS patients. The same inserts used to deliver gancyclovir to AIDS patients are contemplated in this invention.

In yet another preferred embodiment, the eye ointment, the eye shield, the eye wash or the eye drop solution comprises, or is infused with or coated with a CD40:CD154 interruptor that is an anti-CD40L (anti-CD154) compound. In a more preferred embodiment, said anti-CD40L compound is a monoclonal antibody. In yet more preferred embodiments, the monoclonal antibody binds to a protein that is specifically recognized by monoclonal antibody 5c8 produced by ATCC Accession No. HB 10916 or the monoclonal antibody binds to an epitope specifically recognized by monoclonal antibody 5c8 produced by ATCC Accession No. HB 10916.

### Corneal Grafts in Murine Preclinical Studies

The principles of the present invention have been validated by testing in a relevant preclinical model. Corneal transplantation was performed in avascular (normal-risk) or neovascularized (high-risk) eyes of mice (N = 116) randomized to receive anti-CD40L monoclonal antibody and control antibody either by intraperitoneal (i.e., systemic administration) or subconjunctival injection (i.e., local administration). Graft rejection was determined biomicroscopically. Anti-CD40L monoclonal antibody therapy significantly improved survival rates of both normal-risk and high-risk transplants, from rates of 0 - 30% in controls to >90% in anti-CD40L monoclonal antibody treated recipients. Systemic and subconjunctival routes were equally effective. Accordingly, inhibition of the CD40L pathway is a feasible and successful immunomodulatory strategy for preventing corneal transplant rejection in these preclinical studies.

An exemplary CD40:CD154 binding interruptor (the anti-murine-CD40L monoclonal antibody MR1) has been tested alone (monotherapy) in mice transplanted with corneal grafts.

In a preferred embodiment of the invention, the effect of systemic anti-CD40L monoclonal antibody on the outcomes of allogeneic corneal grafts is detailed in **Example 1.** The incidence of corneal allograft rejection was dramatically reduced in recipients with intermittently administered anti-murine-CD40L monoclonal antibody as sole therapy. While treatment with anti-murine-CD40L did not completely prevent graft rejection in minor H-disparate grafts (one graft was lost in this group), the results highlight the important function of the CD40-CD40L pathway in the immunobiology of corneal transplantation, as reflected by the nearly universal acceptance of allografts in hosts receiving anti-murine-CD40L mAb.

The above-described preclinical studies detailed in **Example 1** have been extended to a murine model of corneal graft transplantation in a high-risk host - the inflamed-eye murine model, as detailed in **Example 2.** In that high-risk host model, systemically administered anti-CD40L monoclonal antibody was shown to be equally effective in reducing corneal allograft rejection, as in a normal-risk host model described in **Example 1.**

According to a more preferred embodiment of the present invention, the effect of local (subconjunctival) administration of anti-CD40L monoclonal antibody on the outcome of allogeneic corneal transplant is detailed in **Example 3.**

Anti-CD40L monotherapy has been shown to effectively prevent rejection of cardiac, renal, skin, and other solid organ grafts in both primates and rodents. See, e.g., Kirk et al. (1999), Nature Medicine, 5:686-693. See also Larsen et al. (1996), Nature 381: 434-438. The reported timing and duration of anti-CD40L administration in these studies is variable, ranging from only once perioperatively to once or twice weekly for 2 to 7 weeks followed by once monthly for maintenance. However, in the aggregate, it appears that the initial timing of administration is critical. When treatment is delayed until 5 days postoperatively in the mouse model of cardiac transplantation, no prolongation of graft survival has been observed. See Larsen et al., supra. In the setting of corneal transplantation, a person of skill in the art would appreciate how to determine, without undue experimentation, a schedule with a minimal effective dose and duration of treatment.

It has been shown that the profile of cytokine expression in murine cardiac allografts changes from a Th1-biased (IFN-γ and IL-2) to Th2-biased (IL-4 and IL-10) pattern in anti-CD40L treated animals. See, e.g., Hancock et al. (1996), Proc. Natl. Acad. Sci. USA 93: 13967-12972. Similarly, anti-CD40L mAb may prolong corneal graft survival via suppression of Th1 alloreactive cells. Data herein suggest that induction of Th1 mediated allospecific delayed-type hypersensitivity is suppressed in corneal transplant recipient animals getting anti-CD40L mAb treatment. Since alloreactive responses of the DTH type correlate strongly with graft rejection, anti-CD40L mAb may therefore prolong corneal graft survival via suppression of Th1 alloreactivity. Similarly, it has been shown that the profile of cytokine expression in murine cardiac allografts changes from a Th1-biased (IFN-γ and IL-2) to a Th2-biased (IL-4 and IL-10) pattern in anti-CD40L treated animals. See Hancock WW, Sayegh MH, Zheng XG, Peach R, Linsley PS, Turka LA, Costimulatory function and expression of CD40 ligand, CD80, CD86 in vascularized murine cardiac allograft rejection. Proc. Natl. Acad. Sci. USA.; 93: 13967-13972 (1996).

As shown in **Examples 1-2**, anti-CD40L mAb monotherapy in corneal transplant recipient animals was associated with a blunted postkeratoplasty neovascular response in normal-risk minor H-disparate grafts and in high-risk MHC-disparate grafts during the early postoperative period. This angiostatic action of anti-CD40L mAb may contribute independently to the improved survival of corneal transplants because the presence of corneal neovascularization is a significant risk factor for corneal allograft rejection by serving as a conduit for host immune effector elements.

### MATERIALS AND METHODS FOR EXAMPLES 1 AND 2

### Mice and Anesthesia

Male BALB/c mice aged eight to ten weeks were purchased from Taconic Farms (Germantown, NY) and B10.D2 and BALB.b mice of the same age were purchased from The Jackson Laboratory (Bar Harbor, ME). Prior to all surgical procedures, each animal was deeply anesthetized by intraperitoneal injection of anesthetic agents as described previously (see Sano et al. (1996), Transplant Immunol 4: 53-56). All animals were treated according to the Statement for the Use of Animals in Ophthalmic and Vision Research by the Association for Research in Vision and Ophthalmology.

### Anti-CD40L mAb Administration

Anti-murine-CD40L monoclonal antibody (mAb) (hybridoma MR1, a hamster antibody that specifically binds to murine CD40L), purified from culture supernatant of MR1 hybridoma (ATCC, Rockville, MD) by using a protein A fast-flow column, and control hamster mAb (a non-specific hamster monoclonal antibody) (Biogen, Cambridge, MA) were diluted to a final concentration of 2.5 µg/µl in PBS, aliquoted, and stored at -70°C until use. BALB/c mice were randomly selected to receive either anti-CD40L or hamster mAb at a dose of 250 µg/mouse by intraperitoneal injection. Antibody therapy was given on days -1, 0, 1, and once weekly for weeks 2-7.

### Induction of Corneal Neovascularization ("CNV") in Recipients

Suture-induced CNV is a standardized system of inducing neovascularization ("NV") to create high-risk graft beds. NV growth into the normally avascular corneal stroma can be appreciated from the limbus as early as 3 days after suture placement; neovessels occupy more than 2 quadrants of the central cornea after 14 days. In brief, this model relies on placement of three interrupted sutures (11-0 nylon, 50 mm needle; Sharpoint, Vanguard, Houston, TX) in the central cornea of right eyes of recipient BALB/c mice 14 days prior to corneal transplantation. These mice with neovascularized graft beds then served as high-risk recipients of MHC-disparate orthotopic corneal transplants and the NV-inducing sutures were removed at the time of transplantation.

### Corneal Transplantation

BALB/c mice (N = 78) were used as recipients of syngeneic (BALB/c, N = 10), minor H-disparate (B10.D2, N = 20), or MHC-mismatched (BALB.b, N = 48) corneal transplants. Mice bearing syngeneic grafts received anti-CD40L mAb treatment. All other hosts were randomized to receive either anti-CD40L mAb or control hamster mAb. Syngeneic and minor H-disparate grafts were transplanted to avascular (normal-risk) recipient beds, and MHC-disparate grafts were transplanted to either avascular or neovascularized (high-risk) beds. Corneal transplantation was performed according to well-established protocol (see Sano et al., supra). Briefly on day 0, the central 2 mm area of the donor cornea was excised with Vannas scissors and secured in the host graft bed of 1.8 mm diameter with eight interrupted 11-0 nylon sutures (Sharpoint, Vanguard; Houston, TX). Antibiotic ointment was applied to the corneal surface, and the eyelids were closed for 3 days with a tarsorrhaphy using 8-0 nylon sutures. All grafted eyes were examined 3 days after surgery, and transplant sutures were removed in all mice on day 7.

### Evaluation and Scoring of Orthotopic Corneal Transplants

Grafts were evaluated in a masked fashion for the signs of rejection by slitlamp biomicroscopy twice weekly over eight weeks. At each time point, the grafts were scored for opacity. A previously defined and standardized scoring system was used to grade the degree of opacification from 0 to 5+ (0 = clear graft, 1 = minimal superficial opacity, 2+ = mild stromal opacity with pupil margin and iris vessels visible, 3+ = moderate stromal opacity with only pupil margin visible, 4+ = intense stromal opacity with the anterior chamber visible, 5+ = maximal corneal opacity with total obscuration of the anterior chamber) (see Sano et al., supra). Grafts with an opacity score of 2+ or higher after 3 weeks were considered to be rejected; grafts with an opacity score of 3+ or higher at 2 weeks that never cleared were also regarded as rejected.

### Grading of Postkeratoplasty Corneal Neovascularization

At each time point, the grafts were also scored for neovascularization ("NV"). NV was graded between 0 and 8 based on the degree of centripetal ingrowth and quadrantic involvement of the neovessels under slitlamp biomicroscopy (0 = no vessels, 1+ = vessels in one or two quadrants of recipient bed only, 2+ = vessels in three of four quadrants of recipient bed only, 3+ = vessels at recipient-graft border in one or two quadrants, 4+ = vessels at recipient-graft border in three or four quadrants, 5+ = vessels in peripheral stroma of graft, one or two quadrants, 6+ = vessels in peripheral stroma of graft, three or four quadrants, 7+ = vessels in central stroma of graft, one or two quadrants, 8+ = vessels in central stroma of graft, three or four quadrants). See Sonoda Y, Streilein JW. Orthotopic corneal transplantation in mice--evidence that the immunogenetic rules of rejection do not apply. Transplantation 54:694-704 (1992).

### Assessment of Donor-Specific Delayed-Type Hypersensitivity (DTH)

Because donor-specific DTH can be detected in high-risk recipients bearing fully-mismatched corneal grafts as early as 2 weeks postoperatively, and in normal-risk MHC-disparate grafts 3 weeks postoperatively, we evaluated allospecific DTH responses in high-risk hosts (N = 5 / group) bearing MHC-disparate corneal grafts treated with anti-murine CD40L three weeks after transplantation. The regimen of anti-murine CD40L or hamster control Ig treatment was the same as that aforementioned except that antibody administration was terminated at week 3. Grafted eyes were retained for 3 weeks and then enucleated to cease continuous sensitization by alloantigen. 1 x 10⁶ irradiated (2000 rad) splenocytes in 10 ml of Hanks balanced salt solution from BALB.b donors syngeneic with the corneal graft were injected into the right pinnae, as described previously. BALB/c mice serving as positive controls were immunized by subcutaneous injection of 10 x 10⁶ BALB.b splenocytes one week before ear challenge; BALB/c mice serving as negative controls were only challenged with splenocytes but without prior immunization. At 24 and 48 hours after ear challenge, ear swelling was measured in a masked fashion with a low-pressure micrometer (Mitutoyo, MTI Corp, Paramus, NJ). Ear swelling responses are presented as mean ± SE. Since results at 24 and 48 hours were similar, only 24-hour data are presented.

### statistical Analysis

The rates of corneal graft survival were plotted as Kaplan-Meier survival curves and compared by using the Logrank (Mantel-Cox) test. The difference between CNV scores was analyzed using the nonparametric Wilcoxon signed-rank test. DTH data was presented as mean ± SD and compared by using ANOVA test. Statistical significance was defined as a P value of less than 0.05.

### EXAMPLE 1 (comparative): SYSTEMIC ADMINISTRATION OF ANTI-MURINE-CD40L ANTIBODIES REDUCED INCIDENCE OF CORNEAL GRAFT REJECTION IN NORMAL-RISK MICE

MHC-disparate and minor H-disparate corneas were grafted to BALB/c recipients randomized to receive either hamster mAb, a non-specific hamster monoclonal antibody, or hamster anti-murine-CD40L monoclonal antibody (MR1) therapy. Mice bearing syngeneic BALB/c grafts receiving systemic anti-CD40L mAb served as treatment controls. Syngeneic grafts had a survival rate of 100% at 8 weeks.

### Minor H-Disparate Corneal Grafts

Minor-H disparate grafts treated with hamster mAb exhibited vigorous and prompt rejection events (**Figure 1A**), starting as early as 17 days after transplantation. The cumulative survival rate of this group at 4 and 8 weeks were 63.5% and 25.4% respectively. In contrast, minor-H disparate grafts treated with anti-CD40L mAb displayed significantly increased survival (**Figure 1B**). The only allograft rejection in this group occurred on day 35. The cumulative survival rates in this group at 4 and 8 weeks were 100% and 87%, respectively - significantly higher than rates seen among the hamster mAb-treated control mice (P = 0.0087) (**Figure 1C**). The data demonstrate that systemic anti-CD40L mAb administration can profoundly reduce the rejection rate of minor-H disparate corneal allografts.

Because postkeratoplasty corneal neovascularization (NV) may augment the expression of immunity, the effect of anti-CD40L mAb treatment on corneal neovascularization after transplantation was observed. As shown in **Figure 2**, during the first week after transplantation of minor H-disparate grafts, during the first week after transplantation of minor H-disparate grafts displayed a mean NV score of 2.8 and 2.9 in anti-CD40L mAb treated **(Figure 2B)** and control hosts **(Figure 2A),** respectively. After removal of the corneal sutures at the end of the first week, mean NV scores decreased in both groups. Except for the early time points of week 2 (P = 0.0455) and week 3 (P = 0.0273) at which anti-40L mAb treated hosts displayed a significantly lower mean NV score, the NV scores at the remaining time points were only modestly (P > 0.05) lower in the anti-40L mAb treated hosts. The NV response in syngeneic controls was comparable to that seen in anti-40L mAb treated recipients.

### MHC-Disparate Corneal Grafts

The results of MHC-disparate graft survival (**Figure 3**) showed that 88.9% and 77.8% of MHC-disparate grafts treated with hamster mAb were not rejected at 4 and 8 weeks, respectively (**Figure 3A**). In contrast, grafted hosts treated with anti-CD40L mAb had universal survival of their allogeneic grafts for the entire observation period (**Figure 3B**). However, due to the high rate of acceptance of MHC-disparate grafts among control hosts (see Sano et al. (1996), supra), the increased rate of graft survival among hosts treated with anti-CD40L mAb did not reach statistical significance (P = 0.177) (**Figure 3C**). This high survival of MHC-disparate grafts, even among untreated controls, is consistent with previous data suggesting that minor alloantigens play a more significant role in corneal allograft rejection (see Sano et al. (1996), supra).

There was no difference in the degree of corneal neovascularization between anti-CD154 treated and control animals (P > 0.05 and **Figure 4**).

### EXAMPLE 2 (comparative) : SYSTEMIC ADMINISTRATION OF ANTI-MURINE-CD40L ANTIBODIES REDUCED INCIDENCE OF CORNEAL GRAFT REJECTION IN HIGH-RISK MICE

### High-risk MHC-Disparate Corneal Grafts

Because MHC-disparate corneal grafts transplanted into normal avascular beds have a high acceptance rate even in control animals, the effect of anti-CD40L mAb was further tested on MHC-disparate grafts transplanted into high-risk vascularized beds which exhibit a swift rejection in control hosts. Among control hamster Ig-treated hosts, only 26.8% and 13.4% of grafts survived at 4 and 8 weeks respectively after transplantation, with a majority of the grafts being rejected at 17 days (**Figure 5**). In contrast, anti-CD40L mAb therapy dramatically improved graft survival to 91.7% at both 4 and 8 weeks (P = 0.0002), with only one graft rejected at 21 days (**Figure 5**). The results demonstrate that systemic anti-CD40L mAb treatment can prevent corneal graft rejection in high-risk transplantation.

There was, however, no significant difference in mean NV scores between the two groups at most time points except at week 2 when anti-CD40L mAb treated hosts exhibited lower corneal NV than control hosts (P = 0.0152, **Figure 6**), even though there was enhanced graft survival due to anti-CD40L mAb treatment. Apparently, administration of anti-CD40L mAb does not halt angiogenesis initiated prior to the administration of anti-CD40L mAb; but may be limited to inhibiting neo-angiogenesis.

### Donor-Specific DTH

Donor-specific DTH was evaluated in BALB/c mice after high-risk corneal transplantation. As shown in Figure 7, control mice treated with hamster Ig mounted a vigorous ear swelling response to splenocytes from BALB.b (MHC-disparate) mice. Although not as vigorous as that in DTH positive controls, the allospecific DTH response among grafted animals treated with control Ig was more intense than that in naive animals. In contrast, anti-CD40L mAb treated hosts exhibited a significant decrease in the degree of ear swelling compared with control mice (P < 0.05), suggesting that anti-CD40L mAb therapy suppresses alloreactive CD4⁺ cells of the Th1 type that mediate DTH response.

### EXAMPLE 3: EFFICACY OF LOCALLY ADMINISTERED ANTI-MURINE CD40L MONOCLONAL ANTIBODY ON CORNEAL GRAFT SURVIVAL IN NORMAL-RISK MICE

As shown in Table 1, twenty four normal-risk BALB/c mice received corneal allograft transplant from fully-MHC-mismatched B6 donors. Twelve control group BALB/c transplant recipient mice received a non-specific hamster immunoglobulin ("hamster Ig") in PBS at 50 µg/20 µl/mouse by local administration (subconjunctival injection). Twelve test group BALB/c transplant recipient mice received anti-murine-CD40L monoclonal antibody in PBS at 50 µg/20 µl/mouse by local administration (subconjunctival injection). Mice were treated with hamster Ig or anti-murine-CD40L monoclonal antibody on the day before the transplant (day -1), the day of the transplant (day 0), day 1 post-transplant, and twice per week thereafter.

As shown in **Figure 8**, the cumulative graft survival rate of the test group was 100% after 35 days post-transplant, as compared to approximately 50% for the control group after 35 days post-transplant. After 56 days post-transplant, the cumulative graft survival rate for the test group was still about 90%, as compared to approximately 30% for the control group.

Thus, locally administrated anti-CD40L monoclonal antibody was effective in enhancing corneal allograft survival in normal-risk recipients. Local administration of anti-CD40L mAb was shown to be as effective as systemic administration in Example 1 in the prevention of corneal allograft rejection.

**TABLE 1**

| **Groups** | **Recipient** | **Donor** | **Mice#** | Treatment Schedule (day) |
|---|---|---|---|---|
| | | | | -1. 0, 1; then twice / week thereafter |
| Fully-mismatched control | BALB/c | B6 | 12 | B → |
| Fully-mismatched test | BALB/c | B6 | 12 | A → |

| | | | | |
|---|---|---|---|---|
| A: Anti-CD40L monoclonal antibody in PBS, 50 µg/20 µl/mouse, subconjunctival injection B: Hamster Ig in PBS, 50 µg/20 µl/mouse, subconjunctival injection | | | | |

### Conclusion based on Preclinical Model Studies

The above-described results indicate that induction of corneal graft integration with the CD40:CD154 binding interruptor MR1 (which specifically binds to murine CD40L) alone can lead to long-term survival of corneal allograft in murine preclinical model studies.

## Claims

1. Anti-CD40L (anti-CD154) monoclonal antibody for inhibiting ocular neovascularization in a subject.

2. The anti-CD40L (anti-CD154) monoclonal antibody according to claim 1, for the inhibition of ocular neovascularization that affects a tissue selected from the group consisting of choroidal, corneal, retinal, uveal and iris tissue.

3. The anti-CD40L (anti-CD154) monoclonal antibody according to claim 2, wherein the ocular neovascularization is associated with opacification of said tissue.

4. The anti-CD40L (anti-CD154) monoclonal antibody according to claim 1, wherein said monoclonal antibody specifically binds to a protein to which monoclonal antibody 5c8 produced by ATCC Accession No. HB 10916 specifically binds.

5. The anti-CD40L (anti-CD154) monoclonal antibody according to claim 4, wherein said monoclonal antibody specifically binds to an epitope to which monoclonal antibody 5c8 produced by ATCC Accession No. HB 10916 specifically binds.

6. The anti-CD40L (anti-CD154) monoclonal antibody according to claim 1, wherein said monoclonal antibody is a humanized monoclonal antibody, or a human monoclonal antibody.

7. The anti-CD40L (anti-CD154) monoclonal antibody according to any one of claims 1-6, which is to be administered by means selected from the group consisting of :
(a) parenteral administration ;
(b) biocompatible or bioerodable sustained release implant ;
(c) implantation ofan infusion pump; and
(d) local administration.

8. The anti-CD40L (anti-CD154) monoclonal antibody according to claim 7, wherein the local administration is to be performed by subconjunctival administration.

9. The anti-CD40L (anti-CD154) monoclonal antibody to claim 7, which is to be administered by parenteral administration selected from the group consisting of oral administration, enteral administration and topical administration.

10. The anti-CD40L (anti-CD154) monoclonal antibody according to claim 9, whereby the topical administration is to be performed by means selected from the group consisting of a contact lens, an eye wash solution, an eye ointment, an eye shield and an eye drop solution.

11. The anti-CD40L (anti-CD154) monoclonal antibody according to claim 7, in combination with an immunomodulatory or immunosuppressive compound to be administered to said subject

12. The anti-CD40L (anti-CD154) monoclonal antibody according to claim 11, wherein the immunomodulatory or immunosuppressive compound is a corticosteroid or an antibody that specifically binds to a protein selected from the group consisting of CD45, CD2, IL2R, CD4, CD8 and RANK Fc.

13. The anti-CD40L (anti-CD154) monoclonal antibody according to claim 11, wherein the immunomodulatory or immunosuppressive compound is selected from the group consisting of tacrolimus, rapamycin, mizorubine, deoxyspergualin, brequinar sodium, leflunomide and azaspirane.

14. The anti-CD40L (anti-CD154) monoclonal antibody according to claim 11, wherein the immunomodulatory or immunosuppressive compound is selected from the group consisting of cyclosporin and FK506.

15. The anti-CD40L (anti-CD154) monoclonal antibody according to claim 11, wherein the immunomodulatory or immunosuppressive compound is selected from the group consisting of mycophenolate mofetil and azathioprene.

16. The anti-CD40L (anti-CD154) monoclonal antibody according to any one of claims 1-15, wherein the subject is a mammal.

17. The anti-CD40L (anti-CD154) monoclonal antibody according to claim 16, wherein the mammal is a primate.

18. The anti-CD40L (anti-CD154) monoclonal antibody according to claim 17, wherein the primate is a human.

## Patentansprüche

1. Anti-CD40L (Anti-CD154)-monoclonaler Antikörper für die Hemmung der Neovaskularisation des Auges bei einem Individuum.

2. Anti-CD40L (Anti-CD154)-monoclonaler Antikörper nach Anspruch 1 zur Hemmung einer Neovaskularisation des Auges, die ein Gewebe betrifft, das ausgewählt ist aus der Gruppe bestehend aus Aderhaut-, Hornhaut-, Netzhaut-, Uveo- und Irisgewebe.

3. Anti-CD40L (Anti-CD154)-monoclonaler Antikörper nach Anspruch 2, wobei die Neovaskularisation des Auges mit einer Opakifikation des Gewebes einhergeht.

4. Anti-CD40L (Anti-CD154)-monoclonaler Antikörper nach Anspruch 1, wobei der monoclonale Antikörper spezifisch an ein Protein bindet, an das der von ATCC-Hinterlegungsnummer HB 10916 produzierte monoclonale Antikörper 5c8 spezifisch bindet.

5. Anti-CD40L (Anti-CD154)-monoclonaler Antikörper nach Anspruch 4, wobei der monoclonale Antikörper spezifisch an ein Epitop bindet, an das der von ATCC-Hinterlegungsnummer HB 10916 produzierte monoclonaler Antikörper 5c8 spezifisch bindet.

6. Anti-CD40L (Anti-CD154)-monoclonaler Antikörper nach Anspruch 1, wobei der monoclonale Antikörper ein humanisierter monoclonaler Antikörper oder ein menschlicher monoclonaler Antikörper ist.

7. Anti-CD40L (Anti-CD154)-monoclonaler Antikörper nach einem der Ansprüche 1 bis 6, der über einen Verabreichungsweg verabreicht werden soll, der ausgewählt ist aus der Gruppe bestehend aus:
(a) parenteraler Verabreichung
(b) nachhaltigem, biokompatiblem oder biologisch abbaubarem Implantat mit verzögerter Wirkstofffreisetzung
(c) Implantation einer Infusionspumpe; und
(d) lokaler Verabreichung.

8. Anti-CD40L (Anti-CD154)-monoclonaler Antikörper nach Anspruch 7, wobei die lokale Verabreichung als subkonjunktivale Verabreichung durchgeführt werden soll.

9. Anti-CD40L (Anti-CD154)-monoclonaler Antikörper nach Anspruch 7, der mittels parenteraler Verabreichung verabreicht werden soll, die ausgewählt ist aus der Gruppe bestehend aus oraler, enteraler und topischer Verabreichung.

10. Anti-CD40L (Anti-CD154)-monoclonaler Antikörper nach Anspruch 9, wobei die topische Verabreichung mit Mitteln durchgeführt werden soll, die ausgewählt sind aus der Gruppe bestehend aus Kontaktlinsen, einer Augenspüllösung, einer Augensalbe, einem Augenschutz und einer Augentropfenlösung.

11. Anti-CD40L (Anti-CD154)-monoclonaler Antikörper nach Anspruch 7, in Kombination mit einer immunmodulierenden oder immunsuppressiven Verbindung, die dem Individuum verabreicht werden soll.

12. Anti-CD40L (Anti-CD154)-monoclonaler Antikörper nach Anspruch 11, wobei die immunmodulierende oder immunsuppressive Verbindung ein Corticosteroid oder ein Antikörper ist, das/der spezifisch an ein Protein bindet, das ausgewählt ist aus der Gruppe bestehend aus CD45, CD2, IL2R, CD4, CD8 und RANK Fc.

13. Anti-CD40L (Anti-CD154)-monoclonaler Antikörper nach Anspruch 11, wobei die immunmodulierende oder immunsuppressive Verbindung ausgewählt ist aus der Gruppe bestehend aus Tacrolimus, Rapamycin, Mizorubin, Desoxyspergualin, Brequinar-Natrium, Leflunomid und Azaspiran.

14. Anti-CD40L (Anti-CD154)-monoclonaler Antikörper nach Anspruch 11, wobei die immunmodulierende oder immunsuppressive Verbindung ausgewählt ist aus der Gruppe bestehend aus Cyclosporin und FK506.

15. Anti-CD40L (Anti-CD154)-monoclonaler Antikörper nach Anspruch 11, wobei die immunmodulierende oder immunsuppressive Verbindung ausgewählt ist aus der Gruppe bestehend aus Mycophenolat-Mofetil und Azathiopren.

16. Anti-CD40L (Anti-CD154)-monoclonaler Antikörper nach einem der Ansprüche 1 bis 15, wobei das Individuum ein Säuger ist.

17. Anti-CD40L (Anti-CD154)-monoclonaler Antikörper nach Anspruch 16, wobei der Säuger ein Primat ist.

18. Anti-CD40L (Anti-CD154)-monoclonaler Antikörper nach Anspruch 17, wobei der Primat ein Mensch ist.

## Revendications

1. Anticorps monoclonal anti-CD40L (anti-CD154) pour inhiber une néovascularisation oculaire chez un sujet.

2. Anticorps monoclonal anti-CD40L (anti-CD154) selon la revendication 1 pour l'inhibition d'une néovascularisation oculaire qui affecte un tissu sélectionné dans le groupe consistant en tissu choroïdal, cornéen, rétinien, uvéal et de l'iris.

3. Anticorps monoclonal anti-CD40L (anti-CD154) selon la revendication 2, où la néovascularisation oculaire est associée à une opacification dudit tissu.

4. Anticorps monoclonal anti-CD40L (anti-CD154) selon la revendication 1, où ledit anticorps monoclonal se lie spécifiquement à une protéine à laquelle se lie spécifiquement l'anticorps monoclonal 5c8 produit par ATCC No. d'Accession HB 10916.

5. Anticorps monoclonal anti-CD40L (anti-CD154) selon la revendication 4, où ledit anticorps monoclonal se lie spécifiquement à un épitope auquel se lie spécifiquement l'anticorps monoclonal 5c8 produit par ATCC Accession No. HB 10916.

6. Anticorps monoclonal anti-CD40L (anti-CD154) selon la revendication 1, où ledit anticorps monoclonal est un anticorps monoclonal humanisé ou un anticorps monoclonal humain.

7. Anticorps monoclonal anti-CD40L (anti-CD154) selon l'une quelconque des revendications 1-6, qui doit être administré par un moyen sélectionné dans le groupe consistant en :
(a) administration parentérale ;
(b) implant à libération continue biocompatible ou bioérodable ;
(c) implantation d'une pompe de perfusion ; et
(d) administration locale.

8. Anticorps monoclonal anti-CD40L (anti-CD154) selon la revendication 7, où l'administration locale doit être accomplie par administration subconjonctive.

9. Anticorps monoclonal anti-CD40L (anti-CD154) selon la revendication 7, qui doit être administré par administration parentérale sélectionnée dans le groupe consistant en administration orale, administration entérique et administration topique.

10. Anticorps monoclonal anti-CD40L (anti-CD154) selon la revendication 9, par lequel l'administration topique doit être accomplie par un moyen sélectionné dans le groupe consistant en une lentille de contact, une solution de lavage pour les yeux, un onguent pour les yeux, une protection oculaire et une solution de gouttes pour les yeux.

11. Anticorps monoclonal anti-CD40L (anti-CD154) selon la revendication 7, en combinaison avec un composé immunomodulateur ou immunosuppresseur à administrer audit sujet.

12. Anticorps monoclonal anti-CD40L (anti-CD154) selon la revendication 11, où le composé immunomodulateur ou immunosuppresseur est un corticostéroïde ou un anticorps qui se lie spécifiquement à une protéine sélectionnée dans le groupe consistant en CD45, CD2, IL2R, CD4, CD8 et RANK Fc.

13. Anticorps monoclonal anti-CD40L (anti-CD154) selon la revendication 11, où le composé immunomodulateur ou immunosuppresseur est sélectionné dans le groupe consistant en tacrolimus, rapamycine, mizorubine, desoxyspergualine, bréquinar sodium, leflunomide et azaspirane.

14. Anticorps monoclonal anti-CD40L (anti-CD154) selon la revendication 11, où le composé immunomodulateur ou immunosuppresseur est sélectionné dans le groupe consistant en cyclosporine et FK506.

15. Anticorps monoclonal anti-CD40L (anti-CD154) selon la revendication 11, où le composé immunomodulateur ou immunosuppresseur est sélectionné dans le groupe consistant en mycophénolate mofétil et azathioprène.

16. Anticorps monoclonal anti-CD40L (anti-CD154) selon l'une quelconque des revendications 1-15, où le sujet est un mammifère.

17. Anticorps monoclonal anti-CD40L (anti-CD154) selon la revendication 16, où le mammifère est un primate.

18. Anticorps monoclonal anti-CD40L (anti-CD154) selon la revendication 17, où le primate est un humain.
